# EUROPEAN PATENT SPECIFICATION

(11) **EP 3 607 993 B1**
(45) Date of publication and mention of the grant of the patent: **10.07.2024**
(21) Application number: 18781431.4
(22) Date of filing: 29.03.2018
(51) Int. Cl.: A61M 39/10, A61J 1/20

(54) **CONNECTOR**
VERBINDER
RACCORD

(30) Priority: 04.04.2017 JP 2017074542
(43) Date of publication of application: 12.02.2020
(73) Proprietor: Nipro Corporation, Settsu-shi, Osaka 566-8510 (JP)
(72) Inventor: OGURO, Takeshi, Osaka-shi Osaka 531-8510 (JP); IGUCHI, Yuya, Osaka-shi Osaka 531-8510 (JP); MORITA, Yasuhiro, Osaka-shi Osaka 531-8510 (JP); KANEMURA, Koichi, Osaka-shi Osaka 531-8510 (JP)
(74) Representative: Prüfer & Partner mbB Patentanwälte · Rechtsanwälte
(86) International application number: PCT/JP2018/013200
(87) International publication number: WO 2018/186276

(56) References cited:
- WO-A1-2015/164339
- JP-A- 2005 504 609
- JP-A- 2010 524 626
- JP-A- 2015 526 236
- US-A1- 2005 137 566
- US-A1- 2012 046 636
- US-A1- 2015 297 459
- US-A1- 2016 331 637

## Description

### TECHNICAL FIELD

The present invention relates to a connector.

### BACKGROUND ART

Conventionally, connectors are described in, for example, Japanese National Patent Publication No. 2010-521219 (PTL 1) and Japanese Patent Laying-Open No. 2011-19924 (PTL 2).

### CITATION LIST

### PATENT LITERATURE

PTL 1: Japanese National Patent Publication No. 2010-521219
PTL 2: Japanese Patent Laying-Open No. 2011-19924

### SUMMARY OF INVENTION

### TECHNICAL PROBLEM

According to PTL 1, a first member is arranged to slide with respect to a second member between a secured position in which at least a tip of a piercing member is housed in a protection chamber and an unsecured position in which the tip of the piercing member is arranged outside the protection chamber.

According to PTL 2, a tip of a syringe adapter is constantly provided in a body.

In the structure described in PTL 1, the tip of the needle can be exposed by person's operation. As a result, a person can come into contact with the tip of the needle. According to PTL 2, the tip of the needle is constantly located in the body and a biasing member is provided. Therefore, the needle cannot be connected unless the adapter is pushed in, and thus, injection of a liquid is difficult.

US 2016 / 331 637 A1 describes a medical connector system including a first connector and a second connector. The first connector has a housing, a biasing member, and at least one projection. The second connector has at least one groove for receiving the at least one projection. The proximal end of the second connector is configured to be at least partially disposed within the distal end of the housing of the first connector. Upon application and release of a first set of opposing axial forces applied to the connector system, the first connector is locked to the second connector and, upon application and release of a second set of opposing axial forces, the first connector is released from the second connector. The connectors may include indicators to show when the connector system is in the locked position

US 2015 / 297 459 Aldescribes a syringe adapter includes a housing having a first end and a second end with the first end configured to be secured to a first container, a cannula having a first end and a second end with the second end positioned within the housing, and a collet having a first end and a second end with at least a portion of the collet received within the housing. The collet includes a body defining a passageway, a seal member received by the passageway, and an arcuate, resilient locking member connected to the body of the collet. The collet is movable from a first position where the locking member is open to receive a mating connector to a second position where radially outward movement of the locking member is restricted.

US 2012 / 046 636 A1 describes a connector section for use in a fluid transfer operation, said connector section comprising a hollow cylindrical outer body having a distal shoulder portion radially protruding from said outer body and terminating with an opening through which the proximal end of a fluid transfer component can be inserted for coupling; a closed proximal cap having a central portion comprising connection means protruding proximally from it to connect to the distal end of a fluid transfer apparatus; a needle holder protruding into the interior of said outer body from a central portion of said closed proximal cap for retaining therein at least one conduit comprising a sharp pointed end and further provided with apertures through which fluid is transferred during said fluid transfer operation; and a double membrane seal actuator reciprocably displaceable within the hollow interior of said outer body.

US 2005 / 137 566 A1 describes connector device for placing a first container, such as a liquid container (e.g. flexible container or syringe), in fluid communication with a second container, such as a drug vial. The connector device has a first sleeve having a first end and a second end. The first sleeve has at the first end, a first attaching member adapted to attach to the liquid container. The connector device also has a second sleeve having a first end and a second end. A second attaching member is attached on the second end of the second sleeve and is adapted to attach to the second container. The second attaching member has a sealing member. A piercing member projects within the sleeves for providing a fluid flow path from the first container to the second container. The connector device is movable from the inactivated position to the activated position wherein the second sleeve moves axially with respect to the first sleeve and wherein the piercing member places the first container and the second container in fluid communication. The connector device includes structure for preventing premature activation of the connector device.

The present invention has been made in light of the above-described problem, and an object of the present invention is to provide a connector that makes it difficult for a person to come into contact with a tip of a needle and that facilitates injection of a liquid by connecting another member because a biasing member is not provided.

### SOLUTION TO PROBLEM

The problem is solved by a connector according to claim 1. Further developments of the inventions are indicated in the dependent claims, respectively..

In the connector configured in that way, the first lock portion is provided on the inner circumferential surface of the first connector member, and thus, it is difficult for an operator to touch the first lock member. As a result, unlocking caused by careless operation can be prevented. Furthermore, when the first lock portion engages with the second member to thereby unlock the housing, the housing can be slid to expose the tip of the needle. In this state, the tip of the needle is inserted into the second member, and thus, injection of a liquid using the needle becomes possible.

### ADVANTAGEOUS EFFECTS OF INVENTION

According to the present invention, there can be provided a connector that makes it difficult for a person to come into contact with a tip of a needle and that facilitates injection of a liquid by connecting another member.

### BRIEF DESCRIPTION OF DRAWINGS

Fig. 1 is a side view of a connector according to a first embodiment.
Fig. 2 is a cross-sectional view taken along line II-II in Fig. 1.
Fig. 3 is an enlarged cross-sectional view showing a portion enclosed with III in Fig. 2.
Fig. 4 is a cross-sectional view of the connector before the connector is connected to an access member.
Fig. 5 is a perspective view of a tubular second connector member.
Fig. 6 is a perspective view of a tubular first connector member.
Fig. 7 is a perspective view of a housing that protects a needle tip.
Fig. 8 is a side view of the connector after the connector is connected to the access member.
Fig. 9 is a cross-sectional view taken along line IX-IX in Fig. 8.
Fig. 10 is a cross-sectional view of a portion enclosed with X in Fig. 9.
Fig. 11 is a cross-sectional view taken along line XI-XI in Fig. 9.
Fig. 12 is an enlarged cross-sectional view showing a portion enclosed with XII in Fig. 11.
Fig. 13 is a perspective view of a second lock portion that locks sliding of the first connector member.
Fig. 14 is a side view of the connector in a state where locking by the second lock portion is released.
Fig. 15 is a cross-sectional view taken along line XV-XV in Fig. 14.
Fig. 16 is a cross-sectional view taken along line XVI-XVI in Fig. 15,
Fig. 17 is a cross-sectional view of a portion enclosed with XVII in Fig. 16.
Fig. 18 is a perspective view of the second lock portion that does not lock sliding of the first connector member.
Fig. 19 is a side view of the connector in a state where a needle communicates with the access member.
Fig. 20 is a cross-sectional view taken along line XX-XX in Fig. 19.
Fig. 21 is a cross-sectional view taken along line XXI-XXI in Fig. 20.
Fig. 22 is a side view of the connector that is transferring a liquid.
Fig. 23 is a side view of the connector in a state where communication with the access member is released.
Fig. 24 is a side view of the connector disconnected from the access member.
Fig. 25 is a side view of the connector separated from the access member.

### DESCRIPTION OF EMBODIMENTS

An embodiment of the present invention will be described in detail hereinafter with reference to the drawings. In the embodiment described below, the same or common portions are denoted by the same reference signs in the drawings and description thereof will not be repeated.

### (Configuration of Connector 1, and Access Member 101 and Syringe 201 Attached to Connector 1)

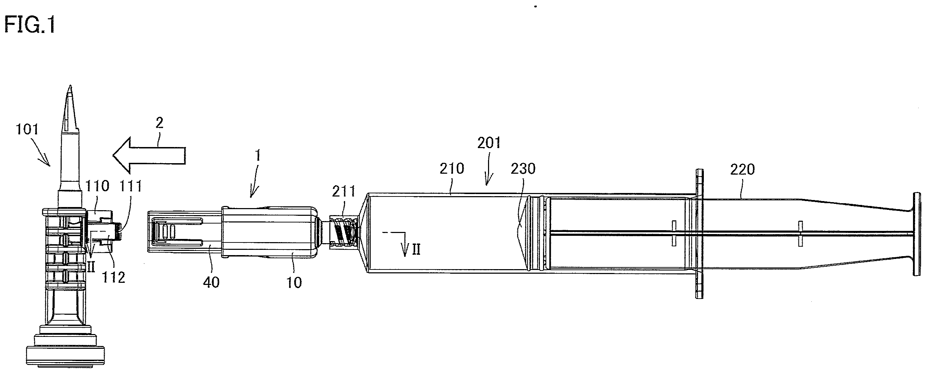

Fig. 1 is a side view of a connector according to a first embodiment. As shown in Fig. 1, a connector 1 has a second connector member 10 serving as a connector main body, and a first connector member 40 serving as a connector guide and fitting into second connector member 10. A tip of first connector member 40 fits into an access member 101. A rear end of second connector member 10 fits into a syringe 201.

Access member 101 has a connector cap 110, and an arm portion 112 held in connector cap 110. A tip of arm portion 112 is a passage pawl portion 111.

Syringe 201 has a circular cylindrical barrel 210, and a plunger 220 that fits into barrel 210. A tip of plunger 220 is fitted with a gasket 230. A tip tube 211 of barrel 210 is screwed onto second connector member 10. Connector 1 moves in a direction indicated by an arrow 2 and the tip of first connector member 40 thereby fits with passage pawl portion 111.

Gasket 230 is provided to be freely slidable in barrel 210 which is a tubular container, and maintains a portion between an inner circumferential surface of barrel 210 and gasket 230 liquid-tight. A liquid 215 is housed in a space formed by barrel 210 and gasket 230. Natural rubber, butyl rubber, chlorinated butyl rubber, ethylene butadiene rubber, thermoplastic elastomer and the like can be used as gasket 230. The space formed by barrel 210 and gasket 230 may be empty such that a drug solution can be housed in the space in use.

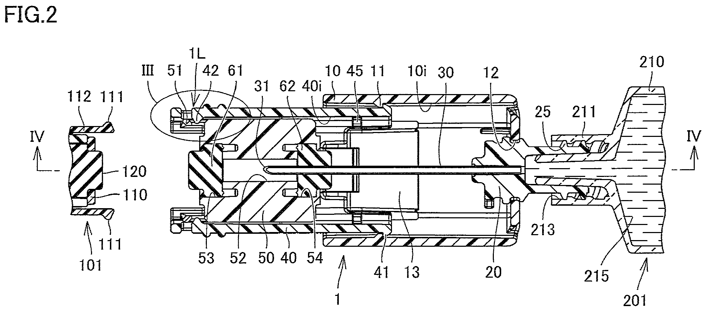

Fig. 2 is a cross-sectional view taken along line II-II in Fig. 1. As shown in Fig. 2, connector 1 has second connector member 10, first connector member 40 that fits into second connector member 10, and a housing 50 that fits into first connector member 40. Second connector member 10 has an inner circumferential surface 10i, An engaging portion 11 is provided on inner circumferential surface 10i. Engaging portion 11 is provided on inner circumferential surface 10i. A through hole 12 is provided at an end of second connector member 10. A hub 20 fits into through hole 12. Second connector member 10 may be integral with hub 20.

A ridge 41 is provided at a rear end of first connector member 40. In a state shown in Fig. 2, ridge 41 engages with engaging portion 11. This restricts movement of first connector member 40 in a direction away from second connector member 10.

First connector member 40 has an inner circumferential surface 40i. A first pawl portion 42 is provided at the tip of first connector member 40. First pawl portion 42 restricts sliding of housing 50. Housing 50 protects a tip 31 of a needle 30. Tip 31 is located in a space 52 which is a through hole of housing 50. A stopper 51 at a tip of housing 50 engages with first pawl portion 42. This restricts sliding of housing 50 in a direction approaching second connector member 10. 50 has a space 53 and a space 54. A second sealing member 61 which is a rubber stopper is housed in space 53. A first sealing member 62 which is a rubber stopper is housed in space 54.

Access member 101 has arm portion 112. The tip of arm portion 112 is passage pawl portion 111. Access member 101 holds a third sealing member 120 which is a rubber stopper. Third sealing member 120 is positioned by connector cap 110.

Hub 20 fits into through hole 12. A male screw 25 is provided at a syringe-side end (female luer lock) of hub 20. Male screw 25 is screwed into a female screw 213 of tip tube (male luer lock) 211 of barrel 210. A component other than syringe 201 may be used as long as connection with hub 20 is possible.

First sealing member 62 and second sealing member 61 may be made of, for example, a rubber member or a thermoplastic elastomer resin. When the rubber member is used, a rubber member represented by isoprene rubber, butyl rubber, silicone rubber and the like can be used.

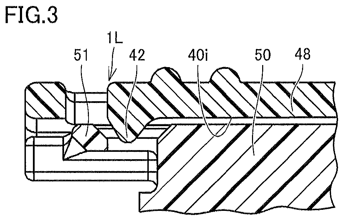

Fig. 3 is an enlarged cross-sectional view showing a portion enclosed with III in Fig. 2. As shown in Fig. 3, first pawl portion 42 and stopper 51 are arranged in an abuttable manner. A first lock portion 1L has first pawl portion 42. In a state shown in Fig. 3, stopper 51 does not abut against first pawl portion 42. However, stopper 51 can slide to a portion that abuts against first pawl portion 42. When stopper 51 abuts against first pawl portion 42, stopper 51 cannot slide in a right direction (direction approaching syringe 201 in Fig. 1) in Fig. 3.

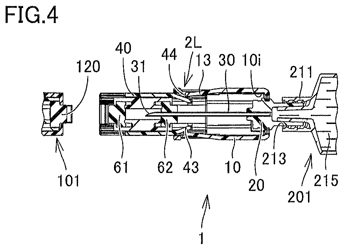

Fig. 4 is a cross-sectional view of the connector before the connector is connected to the access member. As shown in Fig. 4, a first inclined piece 43 is provided at the rear end of first connector member 40. A second inclined piece 13 is provided at a tip of second connector member 10. Second inclined piece 13 and a rear end 44 can abut against each other. Second connector member 10 has a second lock portion 2L. When first connector member 40 slides in the direction approaching second connector member 10, rear end 44 abuts against second inclined piece 13, and sliding of first connector member 40 is thereby restricted.

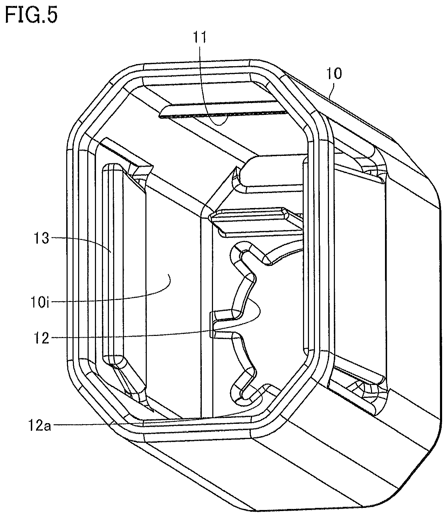

Fig. 5 is a perspective view of the tubular second connector member. As shown in Fig. 5, through hole 12 is provided in the bottom of tubular second connector member 10. In order to allow through hole 12 to bend, through hole 12 is provided with radial grooves 12a. The number of grooves 12a may be larger or smaller than the number shown in Fig. 5. Without having through hole 12, second connector member 10 may be integral with hub 20.

Second inclined piece 13 is provided on inner circumferential surface 10i of second connector member 10 so as to be inclined toward the inner circumferential side. Second inclined piece 13 has flexibility (elasticity). That is, when second inclined piece 13 is pushed outward, second inclined piece 13 moves outward. Engaging portions 11 are provided at two locations of second connector member 10. Second connector member 10 has a tubular shape having a bottom and through hole 12 is provided in the bottom. In this example, second connector member 10 has a substantially octagonal shape. However, the shape of second connector member 10 is not limited thereto, and may be another polygonal shape, a circular shape or an elliptical shape.

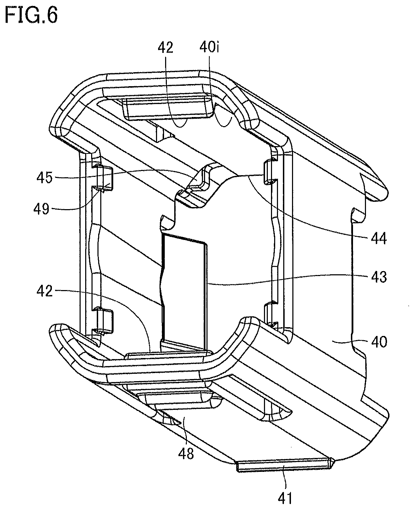

Fig. 6 is a perspective view of the tubular first connector member. First connector member 40 fits with inner circumferential surface 10i of second connector member 10. Since first connector member 40 fits into second connector member 10, first connector member 40 has a shape similar to that of inner circumferential surface 10i of second connector member 10. First pawl portion 42 is provided on inner circumferential surface 40i so as to protrude toward the inner circumferential side. First pawl portion 42 restricts sliding of housing 50. A rear end-side engaging piece 45 is provided on inner circumferential surface 40i. Rear end-side engaging piece 45 serves as a stopper that restricts sliding of housing 50. A front end-side engaging piece 49 is provided on inner circumferential surface 40i. Front end-side engaging piece 49 restricts sliding of housing 50 in a direction away from rear end-side engaging piece 45. First connector member 40 has first inclined piece 43. First inclined piece 43 is a tapered plate member. Ridge 41 is provided on an outer circumferential surface of first connector member 40. Ridge 41 is a convex member extending to be orthogonal to the sliding direction,

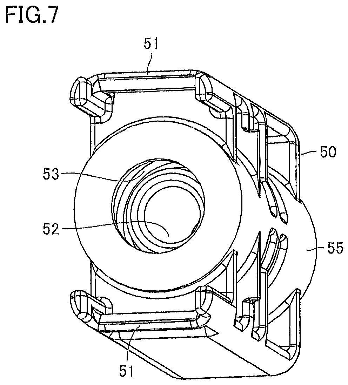

Fig. 7 is a perspective view of the housing that protects the needle tip. As shown in Fig. 7, housing 50 has stoppers 51 provided at the tip portion, a drum-like main body portion 55, space 52 passing through main body portion 55, and space 53 forming an end of space 52. Stoppers 51 extend in a horizontal direction (direction orthogonal to the sliding direction). A pair of stoppers 51 are arranged to be parallel to each other. Main body portion 55 extends from one end to the other end of housing 50. Although main body portion 55 has a circular cylindrical shape in this example, main body portion 55 may have a rectangular cylindrical shape.

### (Operation of Connector 1, and Access Member 101 and Syringe 201 Attached to Connector 1)

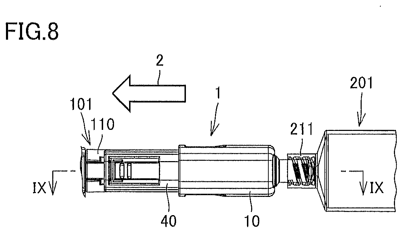

Fig. 8 is a side view of the connector after the connector is connected to the access member. As shown in Fig. 8, the position moves in the direction indicated by arrow 2. As a result, first connector member 40 fits with connector cap 110.

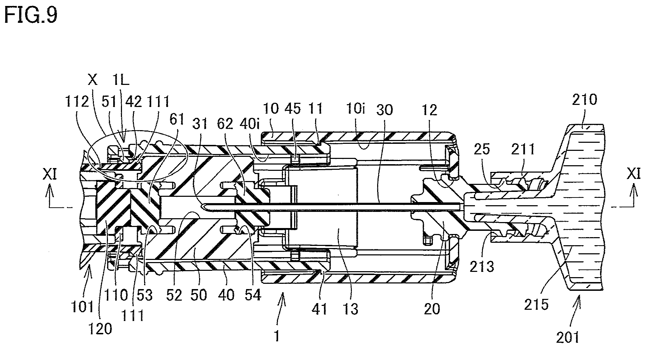

Fig. 9 is a cross-sectional view taken along line IX-IX in Fig. 8. As shown in Fig. 9, second sealing member 61 abuts against third sealing member 120. Passage pawl portion 111 of arm portion 112 abuts against first pawl portion 42 and housing 50. When passage pawl portion 111 pushes housing 50 toward the syringe 201 side, housing 50 tries to move toward the side approaching syringe 201.

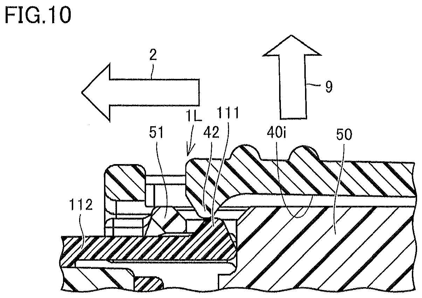

Fig. 10 is a cross-sectional view of a portion enclosed with X in Fig. 9. As shown in Fig. 10, passage pawl portion 111 abuts against first pawl portion 42, and thus, first pawl portion 42 moves upward. Specifically, when a position of first pawl portion 42 shown in Fig. 3 is compared with a position of first pawl portion 42 shown in Fig. 10, the position of first pawl portion 42 shown in Fig. 10 moves upward (in a direction indicated by an arrow 3) relative to the position of first pawl portion 42 shown in Fig. 3.

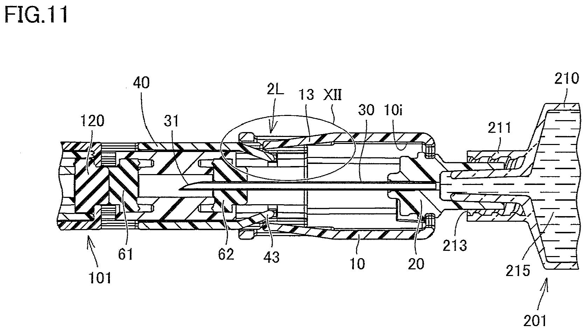

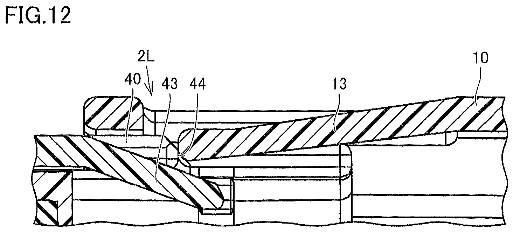

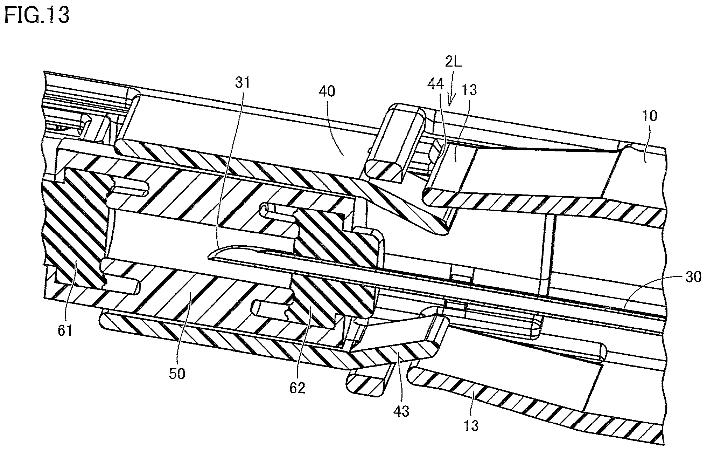

Fig. 11 is a cross-sectional view taken along line XI-XI in Fig. 9. Fig. 12 is an enlarged cross-sectional view showing a portion enclosed with XII in Fig. 11. Fig. 13 is a perspective view of the second lock portion that locks sliding of the first connector member.

As shown in Figs. 11 to 13, a tip of second inclined piece 13 abuts against rear end 44. Therefore, first connector member 40 cannot move from a position shown in Figs. 11 and 12 to a position approaching second connector member 10. Although the force is applied to syringe 201 such that syringe 201 approaches the access member 101 side, first connector member 40 cannot slide and move toward the second connector member 10 side because second lock portion 2L is present.

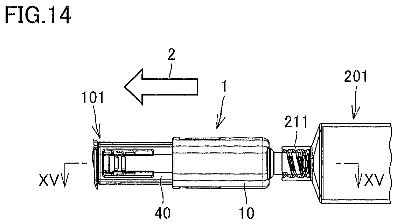

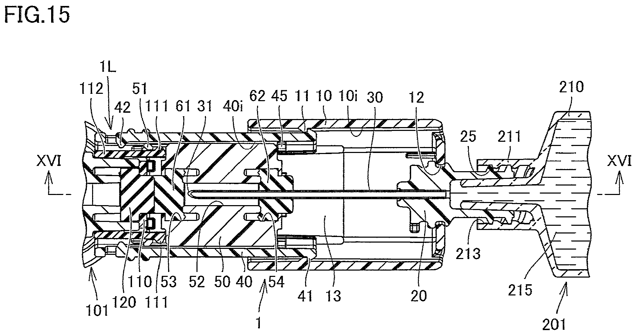

Fig. 14 is a side view of the connector in a state where locking by the second lock portion is released. Fig. 15 is a cross-sectional view taken along line XV-XV in Fig. 14. As shown in Figs. 14 and 15, connector 1 is further moved in the direction approaching access member 101 from the position shown in Fig. 11. Then, first connector member 40 is deeply inserted into access member 101. Stopper 51 abuts against first pawl portion 42, and then, passes first pawl portion 42 and moves in the direction approaching second connector member 10. With this, housing 50 moves in the direction approaching second connector member 10. As a result, a position of tip 31 in space 52 moves and tip 31 approaches second sealing member 61.

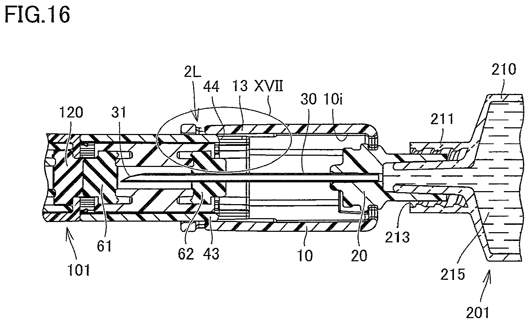

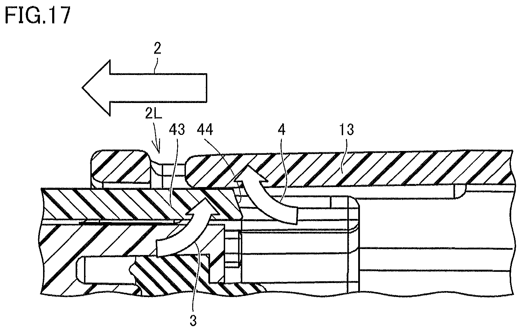

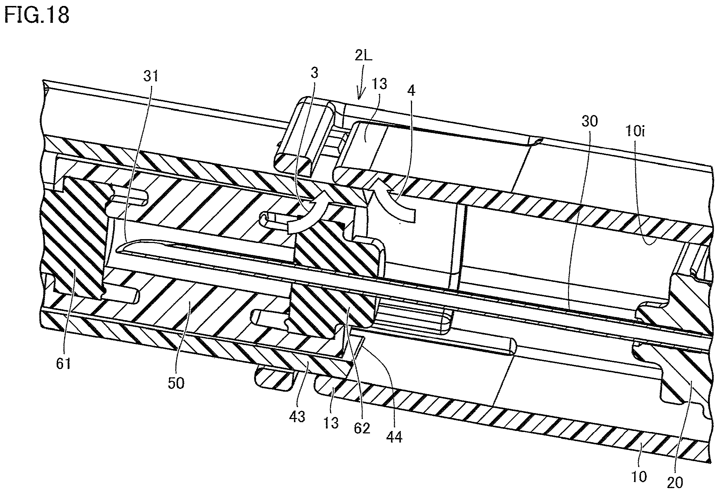

Fig. 16 is a cross-sectional view taken along line XVI-XVI in Fig. 15. Fig. 17 is a cross-sectional view of a portion enclosed with XVII in Fig. 16. Fig. 18 is a perspective view of the second lock portion that does not lock sliding of the first connector member.

As shown in Figs. 16 to 18, housing 50 moves in the direction approaching second connector member 10. As a result, housing 50 abuts against first inclined piece 43 and first inclined piece 43 pivots in the direction indicated by arrow 3. Since an outer circumferential portion of first inclined piece 43 abuts against second inclined piece 13, second inclined piece 13 pivots in a direction indicated by an arrow 4. As a result, first inclined piece 43 and second inclined piece 13 inclined with respect to first connector member 40 and second connector member 10 are no longer inclined with respect to first connector member 40 and second connector member 10 and become parallel to each other. As shown in Fig. 18, second inclined piece 13 is lifted in an outer circumferential direction indicated by an arrow 4. Thus, rear end 44 no longer abuts against second inclined piece 13. As a result, first connector member 40 can slide in the direction approaching second connector member 10.

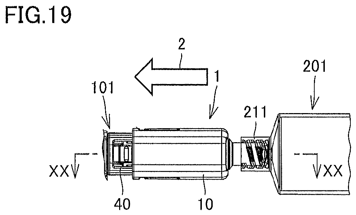

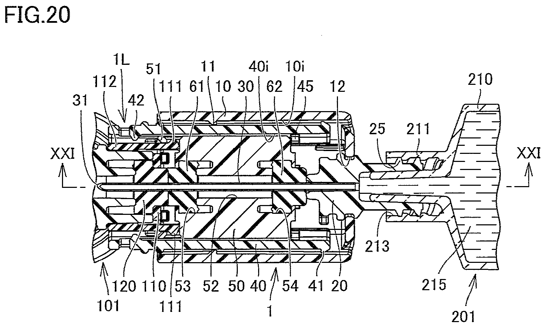

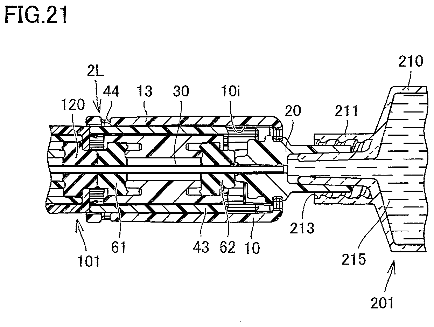

Fig. 19 is a side view of the connector in a state where the needle communicates with the access member. Fig. 20 is a cross-sectional view taken along line XX-XX in Fig. 19. Fig. 21 is a cross-sectional view taken along line XXI-XXI in Fig. 20.

As shown in Figs. 19 to 21, first connector member 40 moves in the direction approaching syringe 201. As a result, first connector member 40 slides in second connector member 10. Housing 50 moves in the direction approaching syringe 201. As a result, tip 31 of needle 30 passes through second sealing member 61 and third sealing member 120 and reaches access member 101. This brings about a state in which liquid 215 can be supplied to access member 101 through needle 30.

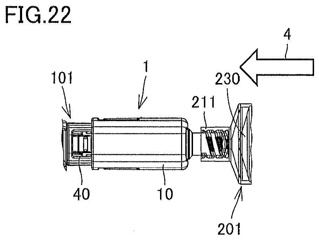

Fig. 22 is a side view of the connector that is transferring the liquid. As shown in Fig. 22, plunger 220 (Fig. 1) is moved in the direction approaching second connector member 10. As a result, liquid 215 in Fig. 21 can be injected into access member 101.

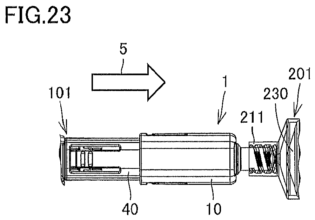

Fig. 23 is a side view of the connector in a state where communication with the access member is released. As shown in Fig. 23, when injection of the liquid ends, it is necessary to detach connector 1 from access member 101. Syringe 201 is moved in a direction indicated by an arrow 5. In a state where injection ends, connector 1 has a structure shown in Fig. 21. When syringe 201 is moved from this state in the direction indicated by arrow 5, second connector member 10 coupled to syringe 201 slides with respect to first connector member 40. As a result, second connector member 10 retracts to a position shown in Figs. 15 to 17.

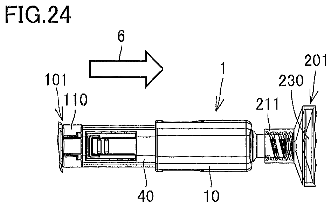

Fig. 24 is a side view of the connector disconnected from the access member. As shown in Fig. 24, when second connector member 10 is further moved in a direction indicated by an arrow 6, first connector member 40 also moves in the direction away from access member 101. As a result, as shown in Figs. 8 to 12, first connector member 40 and second connector member 10 are positioned at the position where second lock portion 2L locks sliding of first connector member 40.

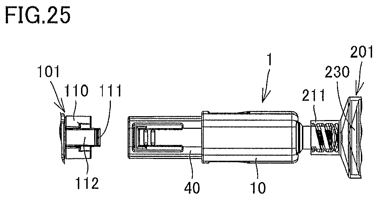

Fig. 25 is a side view of the connector separated from the access member. As shown in Fig. 25, when syringe 201 is moved in the direction away from access member 101, engagement between first connector member 40 and access member 101 is released. As a result, as shown in Fig. 25, connector 1 is separated from access member 101.

Using the connector shown in Fig. 4, a solution in syringe 201 can be injected into a drug vial through connector 1,

When the drug solution is obtained from the drug vial using connector 1, the following procedure is followed.

Connector 1 is connected to syringe 201 (male luer lock syringe). A vial access is connected to the drug vial. Connector 1 is brought close to the vial access and connector 1 is connected to the vial access. When a powdered drug is contained in the drug vial, the solution in syringe 201 can be transferred to the drug vial through connector 1, the powdered drug can be dissolved in the solution, and the solution can be obtained in syringe 201. When a liquid drug is contained in the drug vial, the drug in the drug vial can be obtained in syringe 201 through connector 1.

A method for administering a drug solution using an infusion container in a main route and an infusion container in a sub route will be described. First, an infusion is injected into a patient using the infusion container in the main route and an infusion line. A bag access is connected to the infusion container in the sub route. The bag access is formed by access member 101, connector 1, and a tube that connects access member 101 and connector 1. The drug (drug solution) obtained in the above-described process is present in syringe 201. Connector 1 is connected to access member 101 of the bag access and the drug solution in syringe 201 is transferred from syringe 201 to the bag access. Connector 1 of the bag access is connected to a lock connector in the main route. As a result, the drug solution in the syringe is administered to the patient through the bag access.

### (Effect)

Liquid 215 is contained in syringe 201, and when liquid 215 is any liquid whose leakage to the outside is undesirable, above-described connector 1 has an excellent effect. For example, in addition to dangerous drug such as anti-cancer drug, connector 1 can be used for transferring liquid 215 containing pathogenic bacteria, bacteria to be prevented from having resistance, or the like. Specifically, connector 1 can be appropriately used, for example, for transferring a drug solution from syringe 201 to a drug bag, for transferring an infusion from an infusion line to an empty syringe for sampling, for transferring a liquid specimen containing pathogenic bacteria from a specimen collecting tool to an inspection kit. Also, connector 1 can be used for transferring a dangerous solvent such as trichloroethylene or liquid 215 containing an endocrine-disrupting substance.

Even when such liquid 215 adheres to tip 31 of needle 30, greater safety is secured because tip 31 of needle 30 is not exposed.

Connector 1 is connector 1 interposed between syringe 201 serving as a first member and access member 101 serving as a second member, connector 1 including: needle 30; housing 50 that protects tip 31 of needle 30; tubular first connector member 40 that holds housing 50 in a slidable manner; and first lock portion 1L capable of locking sliding of housing 50, housing 50 protecting tip 31 of needle 30 when sliding of housing 50 and first connector member 40 is locked, first lock portion 1L being provided on inner circumferential surface 40i of first connector member 40, first lock portion 1L engaging with access member 101 to thereby unlock housing 50, and separating from access member 101 to thereby maintain housing 50 locked. First lock portion 1L is provided on inner circumferential surface 40i of first connector member 40, and thus, it is difficult for an operator to touch first lock portion 1L. As a result, unlocking caused by careless operation can be prevented.

Furthermore, when first lock portion 1L engages with access member 101 to thereby unlock housing 50, housing 50 can be slid to expose tip 31 of needle 30. In this state, tip 31 of needle 30 is inserted into the access member, and thus, injection of the liquid using needle 30 becomes possible.

First lock portion 1L has first pawl portion 42 that protrudes toward the inner circumferential surface 40i side of first connector member 40. Since first lock portion 1L is formed by first pawl portion 42, first lock portion 1L can be realized with a simple configuration.

First pawl portion 42 is held by arm portion 48 having elasticity. Since arm portion 48 has elasticity, first pawl portion 42 moves easily when first pawl portion 42 engages with access member 101. As a result, access member 101 engages easily with first pawl portion 42,

Housing 50 is provided with space 52 that houses tip 31 of needle 30 when sliding of housing 50 is locked, housing 50 has first sealing member 62 that seals space 52, and needle 30 passes through first sealing member 62, Since tip 31 of needle 30 is housed in sealed space 52, the liquid does not leak to the outside even when the liquid leaks from tip 31 of needle 30.

Housing 50 has second sealing member 61 that seals space 52, second sealing member 61 is compression-connectable to third sealing member 120 provided in access member 101, and needle 30 passes through second sealing member 61 and third sealing member 120 in a state where second sealing member 61 and third sealing member 120 are compression-connected, and tip 31 of needle 30 thereby reaches access member 101. Since needle 30 passes through second sealing member 61 and third sealing member 120 compression-connected to each other and tip 31 reaches access member 101, exposure of tip 31 of needle 30 to the outside can be prevented. As a result, it is possible to prevent a person from coming into contact with tip 31 of needle 30.

Connector 1 further includes: hub 20 that holds needle 30; tubular second connector member 10 that holds first connector member 40 in a slidable manner; and second lock portion 2L capable of locking sliding of first connector member 40, and through hole 12 that fits with hub 20 is provided in the bottom surface of second connector member 10. Since hub 20 fits into through hole 12, hub 20 can be reliably held by through hole 12. As a result, separation of syringe 201 from connector 1 during transfer of the liquid from syringe 201 through connector 1 to access member 101 can be reduced.

When sliding of housing 50 and first connector member 40 is unlocked and housing 50 and first connector member 40 move in a direction approaching hub 20, first sealing member 62 abuts against hub 20. In this case, hub 20 abuts against first sealing member 62, and thus, hub 20 can be reliably positioned.

First connector member 40 has first inclined piece 43 inclined toward the inner circumferential surface 40i side, and when housing 50 slides toward the first inclined piece 43 side, first inclined piece 43 pivots toward the outer circumferential surface side. Second lock portion 2L has second inclined piece 13 of second connector member 10 located outside first inclined piece 43 and inclined toward the inner circumferential surface 10i side, and when second inclined piece 13 engages with first connector member 40, sliding of first connector member 40 is locked. When first inclined piece 43 pivots outward and abuts against second inclined piece 13 to thereby pivot second inclined piece 13 outward, engagement between second inclined piece 13 and first connector member 40 is released and first connector member 40 becomes slidable. Since sliding of first connector member 40 is locked by first inclined piece 43 inclined toward the inner circumferential surface 40i side and second inclined piece 13 inclined toward the inner circumferential surface 10i side, it is difficult for a person to pivot first inclined piece 43 and second inclined piece 13 outward. As a result, sliding of first connector member 40 caused by person's operation can be reduced.

Access member 101 has passage pawl portion 111 that protrudes toward the outer circumferential side, and passage pawl portion 111 moves first lock portion 1L toward the outer circumferential surface side of first connector member 40 and locking of housing 50 by first lock portion 1L can thereby be released. Housing 50 can be unlocked by operation of first lock portion 1L using passage pawl portion 111, and thus, locking can be released with a simple configuration.

While the embodiment of the present invention has been described above, the embodiment disclosed herein is illustrative and non-restrictive in every respect. The scope of the present invention is defined by the terms of the claims and is intended to include any modifications within the scope and meaning equivalent to the terms of the claims.

### REFERENCE SIGNS LIST

1 connector; 1L first lock portion; 2L second lock portion; 10 second connector member; 10i, 40i inner circumferential surface; 11 engaging portion; 12 through hole; 12a groove; 13 second inclined piece; 20 hub; 25 male screw; 30 needle; 31 tip; 40 first connector member; 41 ridge; 42 first pawl portion; 43 first inclined piece; 44 rear end; 45 rear end-side engaging piece; 48, 112 arm portion; 49 front end-side engaging piece; 50 housing; 51 stopper; 52, 53, 54 space; 55 main body portion; 61 second sealing member; 62 first sealing member; 101 access member; 110 connector cap; 111 passage pawl portion; 120 third sealing member; 201 syringe; 210 barrel; 211 tip tube; 213 female screw; 215 liquid; 220 plunger; 230 gasket.

## Claims

1. A connector (1) interposed between a first member (201) and a second member (101), the connector comprising:
a needle (30);
a hub (20) that holds the needle (30);
a housing (50) that protects a tip (31) of the needle (30);
a tubular first connector member (40) that holds the housing (50) in a slidable manner;
a tubular second connector member (10) that holds the first connector member (40) in a slidable manner; and
a first lock portion (1L) capable of locking sliding of the housing (51),
the hub (20) fitting into a through hole (12) provided in a bottom surface of the second connector member (10) or being formed integral with the second connector member (10),
the first connector member (40) being able to slide in a direction away from the second connector member (10) or in a direction approaching the second connector member (10), the housing (50) being able to slide in a direction away from the second connector member (10) or in a direction approaching the second connector member (10),
the housing (50) protecting the tip (31) of the needle (30) when sliding of the housing (50) and the first connector member (40) is locked,
the first lock portion (1L) being provided on an inner circumferential surface (40i) of the first connector member (40),
the first lock portion (1L) engaging with the second member (101) to thereby unlock the housing (50), and separating from the second member (101) to thereby maintain the housing (50) locked.

2. The connector (1) according to claim 1, wherein
the first lock portion has a first pawl portion (42) that protrudes toward an interior of the first connector member (40).

3. The connector (1) according to claim 2, wherein
the first pawl portion (42) is held by an arm portion having elasticity.

4. The connector according to any one of claims 1 to 3, wherein
the housing (50) is provided with a space (52) that houses the tip of the needle when sliding of the housing (50) is locked,
the housing (50) has a first sealing member (62) that seals the space (52), and
the needle (30) passes through the first sealing member (62).

5. The connector (1) according to claim 4, wherein
the housing (50) has a second sealing member (61) that seals the space (52), the second sealing (61) member is compression-connectable to a third sealing member (120) provided in the second member (101), and
the needle (30) passes through the second sealing member (61) and the third sealing member (120) in a state where the second sealing member (61) and the third sealing member (120) are compression-connected, and the tip (31) of the needle (30) thereby reaches the second member (101).

6. The connector according to claim 4 or 5, further comprising:
a hub (20) that holds the needle (30); and
a second lock portion capable of locking sliding of the first connector member (40), wherein
a through hole (12) that fits with the hub (20) is provided in a bottom surface of the second connector member (10).

7. The connector (1) according to claim 6, wherein
when sliding of the housing (50) and the first connector member (40) is unlocked and the housing (50) and the first connector member (40) move in a direction approaching the hub (20), the first sealing member (62) abuts against the hub (20).

8. The connector (1) according to claim 6 or 7, wherein
the first connector member (40) has a first inclined piece (43) inclined toward an inner circumferential surface side (40i), and when the housing slides (50) toward the first inclined piece side (43), the first inclined piece (43) pivots toward an outer circumferential surface side,
the second lock portion (2L) has a second inclined piece (13) of the second connector member (10) located outside the first inclined piece (43) and inclined toward the inner circumferential surface side (10i), and when the second inclined piece (13) engages with the first connector member (40), sliding of the first connector member (40) is locked, and
when the first inclined piece (43) pivots outward and abuts against the second inclined piece (13) to thereby pivot the second inclined piece (13) outward, engagement between the second inclined piece (13) and the first connector member (40) is released and the first connector member (40) becomes slidable.

9. The connector (1) according to any one of claims 1 to 8, wherein
the second member (10) has a passage pawl portion (111) that protrudes toward an outer circumferential side, and
the passage pawl portion (111) moves the first lock portion (43) toward an outer circumferential surface side of the first connector member (43) and locking of the housing (50) by the first lock portion (1L) can thereby be released.

## Patentansprüche

1. Verbindungsteil (1), das zwischen einem ersten Element (201) und einem zweiten Element (101) angeordnet ist, wobei das Verbindungsteil umfasst:
eine Nadel (30);
eine Nabe (20), die die Nadel (30) hält;
ein Gehäuse (50), das eine Spitze (31) der Nadel (30) schützt;
ein röhrenförmiges erstes Verbindungselement (40), das das Gehäuse (50) verschiebbar hält;
ein röhrenförmiges zweites Verbindungselement (10), das das erste Verbindungselement (40) verschiebbar hält; und
einen ersten Verriegelungsabschnitt (1L), der in der Lage ist, das Verschieben des Gehäuses (51) zu verriegeln,
wobei die Nabe (20) in ein Durchgangsloch (12) passt, das in einer Bodenfläche des zweiten Verbindungselements (10) vorgesehen ist, oder integral mit dem zweiten Verbindungselement (10) ausgebildet ist,
das erste Verbindungselement (40) in einer Richtung weg von dem zweiten Verbindungselement (10) oder in einer Richtung hin zu dem zweiten Verbindungselement (10) verschiebbar ist, wobei das Gehäuse (50) in einer Richtung weg von dem zweiten Verbindungselement (10) oder in einer Richtung hin zu dem zweiten Verbindungselement (10) verschiebbar ist,
das Gehäuse (50) die Spitze (31) der Nadel (30) schützt, wenn das Verschieben des Gehäuses (50) und des ersten Verbindungselements (40) verriegelt ist,
der erste Verriegelungsabschnitt (1L) an einer Innenumfangsfläche (40i) des ersten Verbindungselements (40) vorgesehen ist,
wobei der erste Verriegelungsabschnitt (1L) mit dem zweite Element (101) in Eingriff gelangt, um dadurch das Gehäuse (50) zu entriegeln, und sich von dem zweiten Element (101) trennt, um dadurch das Gehäuse (50) verriegelt zu halten.

2. Verbindungsteil (1) nach Anspruch 1, wobei
der erste Verriegelungsabschnitt einen ersten Sperrklinkenabschnitt (42) aufweist, der in Richtung eines Innenraums des ersten Verbindungselements (40) vorsteht.

3. Verbindungsteil (1) nach Anspruch 2, wobei
der erste Sperrklinkenabschnitt (42) durch einen Armabschnitt mit Elastizität gehalten wird.

4. Verbindungsteil nach einem der Ansprüche 1 bis 3, wobei
das Gehäuse (50) mit einem Raum (52) versehen ist, der die Spitze der Nadel aufnimmt, wenn das Verschieben des Gehäuses (50) verriegelt ist,
das Gehäuse (50) ein erstes Dichtungselement (62) aufweist, das den Raum (52) abdichtet, und
sich die Nadel (30) durch das erste Dichtungselement (62) erstreckt.

5. Verbindungsteil (1) nach Anspruch 4, wobei
das Gehäuse (50) ein zweites Dichtungselement (61) aufweist, das den Raum (52) abdichtet, das zweite Dichtungselement (61) mit einem dritten Dichtungselement (120), das in dem zweiten Element (101) vorgesehen ist, unter Zusammendrücken verbindbar ist, und
sich die Nadel (30) durch das zweite Dichtungselement (61) und das dritte Dichtungselement (120) in einem Zustand erstreckt, in dem das zweite Dichtungselement (61) und das dritte Dichtungselement (120) unter Zusammendrücken miteinander verbunden sind, und die Spitze (31) der Nadel (30) dadurch das zweite Element (101) erreicht.

6. Verbindungsteil nach Anspruch 4 oder 5, weiter umfassend
eine Nabe (20), die die Nadel (30) hält; und
einen zweiten Verriegelungsabschnitt, der in der Lage ist, das Verschieben des ersten Verbindungselements (40) zu verriegeln, wobei
ein Durchgangsloch (12), das zu der Nabe (20) passt, in einer Bodenfläche des zweiten Verbindungselements (10) vorgesehen ist.

7. Verbindungsteil (1) nach Anspruch 6, wobei
wenn das Verschieben des Gehäuses (50) und des ersten Verbindungselements (40) entriegelt ist und das Gehäuse (50) und das erste Verbindungselement (40) sich in eine Richtung zu der Nabe (20) hin bewegen das erste Dichtungselement (62) gegen die Nabe (20) stößt.

8. Verbindungsteil (1) nach Anspruch 6 oder 7, wobei
das erste Verbindungselement (40) ein erstes geneigtes Teil (43) aufweist, das in Richtung einer Innenumfangsflächenseite (40i) geneigt ist, und wenn sich das Gehäuse (50) in Richtung der ersten geneigten Teilseite (43) verschiebt, schwenkt das erste geneigte Teil (43) in Richtung einer Außenumfangsflächenseite,
der zweite Verriegelungsabschnitt (2L) ein zweites geneigtes Teil (13) des zweiten Verbindungselements (10) aufweist, das außerhalb des ersten geneigten Teils (43) angeordnet und in Richtung der Innenumfangsflächenseite (10i) geneigt ist, und wenn das zweite geneigte Teil (13) mit dem ersten Verbindungselement (40) in Eingriff ist, ist das Verschieben des ersten Verbindungselements (40) verriegelt, und
wenn das erste geneigte Teil (43) nach außen schwenkt und gegen das zweite geneigte Teil (13) stößt, um dadurch das zweite geneigte Teil (13) nach außen zu schwenken, wird der Eingriff zwischen dem zweiten geneigten Teil (13) und dem ersten Verbindungselement (40) gelöst und das erste Verbindungselement (40) wird verschiebbar.

9. Verbindungsteil (1) nach einem der Ansprüche 1 bis 8, wobei
das zweite Element (10) einen Durchgangssperrklinkenabschnitt (111) aufweist, der in Richtung einer Außenumfangsseite vorsteht, und
der Durchgangssperrklinkenabschnitt (111) den ersten Verriegelungsabschnitt (43) in Richtung einer Außenumfangsflächenseite des ersten Verbindungselements (43) bewegt und die Verriegelung des Gehäuses (50) durch den ersten Verriegelungsabschnitt (1L) dadurch gelöst werden kann.

## Revendications

1. Un connecteur (1) interposé entre un premier élément (201) et un deuxième élément (101), le connecteur comprenant :
une aiguille (30) ;
un raccord (20) qui maintient l'aiguille (30) ;
un boîtier (50) qui protège une pointe (31) de l'aiguille (30) ;
un premier élément de connecteur tubulaire (40) qui maintient le boîtier (50) de manière coulissante ;
un deuxième élément de connecteur tubulaire (10) qui maintient le premier élément de connecteur (40) de manière coulissante ; et
une première portion de verrouillage (1L) capable de verrouiller le coulissement du boîtier (51),
le raccord (20) s'adaptant dans un trou traversant (12) fourni dans une surface inférieure du deuxième élément de connecteur (10) ou étant formé intégralement avec le deuxième élément de connecteur (10),
le premier élément de connecteur (40) étant capable de coulisser dans une direction s'éloignant du deuxième élément de connecteur (10) ou dans une direction s'approchant du deuxième élément de connecteur (10),
le boîtier (50) étant capable de coulisser dans une direction s'éloignant du deuxième élément de connecteur (10) ou dans une direction s'approchant du deuxième élément de connecteur (10),
le boîtier (50) protégeant la pointe (31) de l'aiguille (30) lorsque le coulissement du boîtier (50) et du premier élément de connecteur (40) est verrouillé,
la première portion de verrouillage (1L) étant fournie sur une surface circonférentielle intérieure (40i) du premier élément de connecteur (40),
la première portion de verrouillage (1L) venant en prise avec le deuxième élément (101) pour déverrouiller ainsi le boîtier (50), et se séparant du deuxième élément (101) pour maintenir ainsi le boîtier (50) verrouillé.

2. Le connecteur (1) selon la revendication 1, dans lequel
la première portion de verrouillage a une première portion de cliquet (42) qui fait saillie vers un intérieur du premier élément de connecteur (40).

3. Le connecteur (1) selon la revendication 2, dans lequel
la première portion de cliquet (42) est maintenue par une portion de bras ayant de l'élasticité.

4. Le connecteur selon l'une quelconque des revendications 1 à 3, dans lequel
le boîtier (50) est pourvu d'un espace (52) qui loge la pointe de l'aiguille lorsque le coulissement du boîtier (50) est verrouillé,
le boîtier (50) a un premier élément d'étanchéité (62) qui scelle l'espace (52), et
l'aiguille (30) passe à travers le premier élément d'étanchéité (62).

5. Le connecteur (1) selon la revendication 4, dans lequel
le boîtier (50) a un deuxième élément d'étanchéité (61) qui scelle l'espace (52), le deuxième élément d'étanchéité (61) est connectable par compression à un troisième élément d'étanchéité (120) prévu dans le deuxième élément (101), et
l'aiguille (30) passe à travers le deuxième élément d'étanchéité (61) et le troisième élément d'étanchéité (120) dans un état où le deuxième élément d'étanchéité (61) et le troisième élément d'étanchéité (120) sont connectés par compression, et la pointe (31) de l'aiguille (30) atteint ainsi le deuxième élément (101).

6. Le connecteur selon la revendication 4 ou 5, comprenant en outre :
un raccord (20) qui maintient l'aiguille (30) ; et
une deuxième portion de verrouillage capable de verrouiller le coulissement du premier élément de connecteur (40), dans lequel
un trou traversant (12) qui s'accorde avec le raccord (20) est fourni dans une surface inférieure du deuxième élément de connecteur (10).

7. Le connecteur (1) selon la revendication 6, dans lequel,
lorsque le boîtier (50) coulisse, et le premier élément de connecteur (40) est déverrouillé et le boîtier (50), et le premier élément de connecteur (40) se déplacent dans une direction s'approchant du raccord (20), le premier élément d'étanchéité (62) vient en butée contre le raccord (20).

8. Le connecteur (1) selon la revendication 6 ou 7, dans lequel
le premier élément de connecteur (40) a une première pièce inclinée (43) qui est inclinée vers un côté de surface circonférentielle intérieure (40i), et lorsque le boîtier (50) coulisse vers le côté de première pièce inclinée (43), la première pièce inclinée (43) pivote vers un côté de surface circonférentielle extérieure,
la deuxième portion de verrouillage (2L) a une deuxième pièce inclinée (13) du deuxième élément de connecteur (10) située à l'extérieur de la première pièce inclinée (43) et inclinée vers le côté de surface circonférentielle intérieure (10i), et lorsque la deuxième pièce inclinée (13) vient en prise avec le premier élément de connecteur (40), le coulissement du premier élément de connecteur (40) est verrouillé, et
lorsque la première pièce inclinée (43) pivote vers l'extérieur et vient en butée contre la deuxième pièce inclinée (13) pour ainsi faire pivoter la deuxième pièce inclinée (13) vers l'extérieur, l'engagement entre la deuxième pièce inclinée (13) et le premier élément de connecteur (40) est libéré et le premier élément de connecteur (40) devient coulissant.

9. Le connecteur (1) selon l'une quelconque des revendications 1 à 8, dans lequel
le deuxième élément (10) a une portion de cliquet de passage (111) qui fait saillie vers un côté circonférentiel extérieur, et
la portion de cliquet de passage (111) déplace la première portion de verrouillage (43) vers un côté de surface circonférentielle extérieure du premier élément de connecteur (43) et le verrouillage du boîtier (50) par la première portion de verrouillage (1L) peut ainsi être libéré.
